# EUROPÄISCHE PATENTSCHRIFT

(11) **EP 0 982 075 B1**
(45) Veröffentlichungstag und Bekanntmachung des Hinweises auf die Patenterteilung: **02.07.2008**
(21) Anmeldenummer: 99121618.5
(22) Anmeldetag: 27.07.1995
(51) Int. Cl.: B05B 11/00, A61F 9/00

(54) **Austragvorrichtung für Medien**
Dispensing device for fluids
Distributeur de fluides

(30) Priorität: 05.08.1994 DE 9412662 U
(43) Veröffentlichungstag der Anmeldung: 01.03.2000
(62) Teilanmeldung aus: 95111795.1
(73) Patentinhaber: Ing. Erich Pfeiffer GmbH, 78315 Radolfzell (DE)
(72) Erfinder: Fuchs, Karl Heinz, 78315 Radolfzell (DE)
(74) Vertreter: Patentanwälte Ruff, Wilhelm, Beier, Dauster & Partner

(56) Entgegenhaltungen:
- GB-A- 2 142 829
- US-A- 2 058 515
- US-A- 2 898 911
- US-A- 3 841 533
- US-A- 5 064 420

## Beschreibung

Die Erfindung betrifft eine Austragvorrichtung für Medien flüssigen, pastösen, pulverförmigen und/oder gasförmigen Aggregatzustandes während und/oder nach dem Austrag. Die Austragvorrichtung kann nur durch einen ein- oder mehrteiligen Austragkopf aus ggf. untereinander nicht bewegbaren bzw. miteinander einteiligen Bauteilen oder durch eine Einheit gebildet sein, welche jeweils ein- oder mehrfach einen Medienauslaß, einen Austragförderer, eine Austragbetätigung und/oder einen Medienspeicher aufweist. Der Austragkopf kann mit einer einfachen, in einer einzigen Achse liegenden Verbindung aus Dorn und Hülse oder dgl. mit einem der genannten Bauteile der vormontierten Einheit lagestarr oder zur Betätigung bewegbar sowie ggf. lösbar zu verbinden sein. Der Austragkopf ist dadurch dann an eine Medien-Auslaßöffnung dieses Bauteiles angeschlossen.

Für medizinische, kosmetische, technische und ähnlich Anwendungen ist es häufig zweckmäßig, den mit dem Medium zu beaufschlagenden Teil, z.B. einen menschlichen oder tierischen Körperbereich bzw. eine Oberfläche oder Deckschicht in einen Zustand zu versetzen oder in diesem zu halten, um dadurch die Medienbeaufschlagung zu optimieren. Der Zustand kann ein bestimmter Radial- und/oder Axialabstand zum Medienauslaß, eine Straffung, Vorspannung oder Zusammenziehung der Oberfläche bzw. Deckschicht, deren begrenzte Belüftung, Beleuchtung bzw. Abdunkelung, ein Anschluß der Oberfläche an eine Kammer zur Rückhaltung des Mediums, insbesondere vernebelten Mediums, eine Umfangsabdichtung der zu beaufschlagenden Oberflächenzone oder dgl. sein.

Aus der EP 0 347 084 A ist ein Augentropfer bekannt geworden, bei dem eine Quetschflasche mit einem ovalen, tassenförmigen Adapter versehen ist. Er hat eine ovale Grundfläche und besteht aus elastisch verformbarem Material. Er wird vor dem Aufsetzen auf das Auge von Hand unter Reduzierung seines Volumens zusammengedrückt und weitet sich aufgrund seiner Elastizität beim Aufsetzen auf das Auge wieder auf sein ursprüngliches Volumen auf.

Aus der GB 2 142 829 A ist ein Augentropfer ähnlicher Art bekannt geworden, der eine ovale ringförmige Anlagefläche an einem Adapter aufweist, die über schräge Stege mit der Aufnahme für die Tropfflasche verbunden sind. Dieser Adapter besteht aus formstarrem Material.

Die US 3,841,533 A beschreibt einen Adapter für den Sprühkopf einer Aerosolflasche, der im Ruhezustand mit seiner trichterförmigen Adapterkammer auf den Sprühkopf aufgesteckt ist und zur Benutzung abgezogen und in anderer Lage auf den Sprühkopf aufgesteckt werden kann. Er hat eine starre, trichterförmige Sprühkammer.

Die US 2 058 515 A (vgl. Oberbegriff des ersten Anspruchs) beschreibt einen Augen-Spülbecher mit einem tassenförmigen Adapter, der eine an das Auge angepaßte Form hat. Er kann aus elastischem, flexiblem Material bestehen, um sich besser an das Auge anpassen zu können.

Der Erfindung liegt die Aufgabe zugrunde, eine Austragvorrichtung für Medien zu schaffen, durch welche Nachteile bekannter Ausbildungen vermieden bzw. Vorteile der genannten Art zu erreichen sind, wobei insbesondere eine baulich sehr einfache Ausbildung die jeweilige Zustandsbeeinflussung des zur beaufschlagenden Teiles, besonders eine Straffung, Vorspannung etc. einer Körperoberfläche, ermöglichen soll.

Die Aufgabe wird durch die Merkmale des Anspruchs 1 gelöst. Durch die Aufweitung der Breitseiten der langgestreckten ringförmigen Anlagefläche verändert sich dessen Form und Flächengröße, was eine wirksame Straffung des Teiles auf die die Zerstäuberdüse das Medium sprüht, z.B. der Haut bewirkt. Das Arbeitsglied kann in Richtung des Austragstromes des Mediums so vorgelagert sein, dass es diesen Medienstrom nicht oder allenfalls nur geringfügig beeinflusst. Dadurch kann der Medienstrom, z.B. ein feinstzerstäubter Sprühstrahl, ein gebündelter bzw. unaufgefächerter Strahl vom Medienauslaß unmittelbar der zu beaufschlagenden Oberfläche zugeführt werden, welche ihrerseits durch das Arbeitsglied gestrafft, vorgespannt, begrenzt etc. ist. Zur Beeinflussung bzw. Herstellung des jeweiligen Zustandes können gesonderte Arbeitsglieder gegenüber dem Medienauslaß oder der genannten Baueinheit auswechselbar bzw. in gleichzeitiger mehrfacher Anordnung vorgesehen sein oder es kann ein einziges Arbeitsglied mehrere der genannten Zustände herstellen.

Das Arbeitsglied gibt eine an den Medienauslaß angeschlossene Kammer mit einer Anlagefläche für den zu beaufschlagenden Teil, so daß die Kammer nach Anlage an diesen Teil zumindest über einen Teilumfang und/oder eine Teillänge ihres zugehörigen Endes geschlossen ist und Medium hier radial nicht austreten kann. Die zugehörige Kammeröffnung kann den weitesten Innenbereich der Kammer bilden, welche zur Kammeröffnung zweckmäßig annähernd über ihre gesamte Länge bzw. etwa vom Medienauslaß bis zur Kammeröffnung durchgehend stetig und/oder stufenförmig erweitert ist. Zur ventilfreien Belüftung der Kammer kann deren einzige Mantel- bzw. Bodenwand mit einem oder mehreren Öffnungen versehen sein, über welche auch bei einem Druckstoß ein sofortiger Druckausgleich mit dem atmosphärischen Druck der Umgebung erfolgt. Des weiteren kann durch die jeweilige Öffnung direkter Einblick auf die zu beaufschlagende Oberflächenzone von außen genommen werden, wenn bereits der genannte Zustand hergestellt ist.

Das Arbeitsglied ist teilweise in sich, insbesondere rückfedernd elastisch, verformbar, vorzugsweise im Bereich seiner vorderen Anlagefläche, seines Mantels und/oder seiner Bodenwandung. Zur Erhöhung der Elastizität kann es in Teilbereichen in seinen Materialquerschnitten geschwächt, z.B. mit Vertiefungen, Öffnungen oder über den Umfang geschlossenen Durchbrüchen versehen sein, so daß es z.B. steg-, platten-, laschen- oder armförmige Biegeglieder bildet, welche im Querschnitt gleich oder unterschiedlich gekrümmt sein können bzw. in der Hüllfläche der in Umfangsrichtung anschließenden Mantelwand liegen. Ein Träger für das Arbeitsglied, insbesondere der Austragkopf, kann demgegenüber dadurch wesentlich formsteifer bzw. hinsichtlich der Betriebsbelastungen formstarr ausgebildet sein, daß er wesentlich dickere Materialquerschnitte als das Arbeitsglied aufweist. Dadurch kann ein formsteifer Träger einteilig mit einem Bauteil, insbesondere dem Arbeitsglied, ausgebildet sein, welches für den Betrieb wenigstens teilweise in einer oder mehreren zueinander rechtwinkligen Richtungen verformbar bzw. gegenüber dem Träger lageveränderbar ist.

Der durch eine Schubkolbenpumpe gebildete Austragförderer kann dabei zum nur einmaligen Austrag einer einzigen Mediendosis oder dafür vorgesehen sein, gleiche Mediendosen wiederholt z.B. dadurch auszubringen, daß die Austragpumpe aus einem fest mit ihr verbundenen Speichergefäß nach jedem Austrag wieder Medium selbsttätig nachsaugt.

Die erfindungsgemäße Ausbildung ist insbesondere zur Schaffung eines Augensprays geeignet, indem z.B. das Arbeitsglied eine um den Rand der Augenhöhle anzusetzende Augenschale bildet und im angesetzten Zustand der Medienauslaß gegen den Augapfel gerichtet ist. Das obere und/oder untere Augenlid kann durch das Ansetzen im Sinne einer Öffnung bewegt bzw. in seiner Lage gesichert werden, so daß ein reflexartiges Schließen des Auges aufgrund des ausgestossenen Medienstrahles verhindert ist. Gleichzeitig wird ein genau definierter Abstand zwischen Medienauslaß und Augapfel bzw. Pupille festgelegt. Begrenzte Zonen des Lichteinfalles in die Schale können bei einer bestimmten Ausrichtung des Augapfels als Blickpunkte mitwirken.

Diese und weitere Merkmale gehen außer aus den Ansprüchen auch aus der Beschreibung und den Zeichnungen hervor. Ein Ausführungsbeispiel der Erfindung ist in den Zeichnungen dargestellt und wird im folgenden näher erläutert. In den Zeichnungen zeigen:

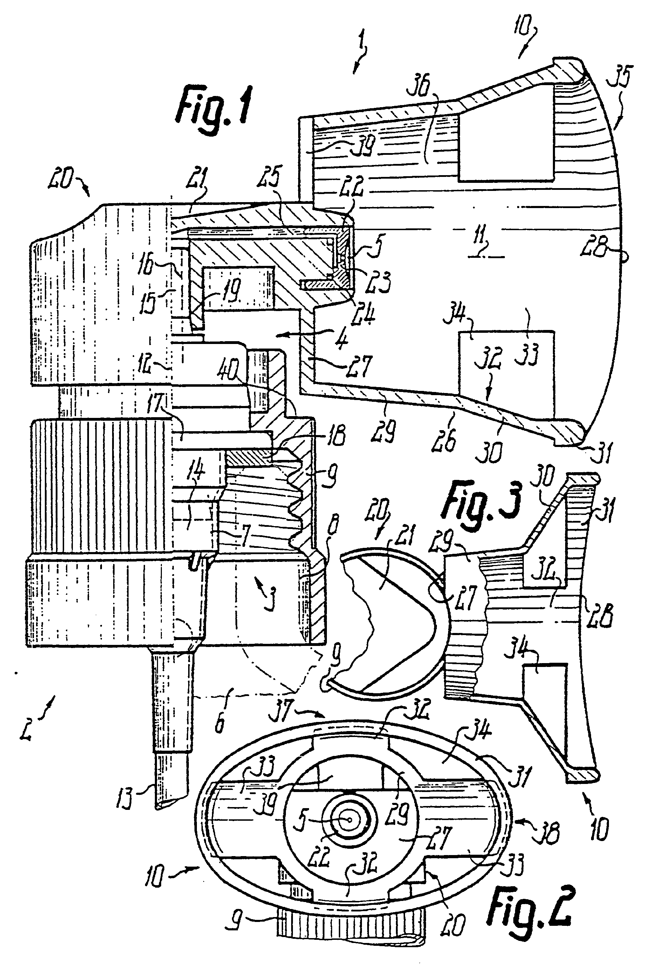

- Fig. 1: eine erfindungsgemäße Austragvorrichtung in teilweise geschnittener Ansicht,
- Fig. 2: die Austragvorrichtung gemäß Fig. 1 in Axialansicht und
- Fig. 3: die Austragvorrichtung gemäß Fig. 1 in Ansicht von oben.

Die Austragvorrichtung 1 ist Bestandteil einer vormontierten Austrageinheit 2, welche jeweils in einfacher oder mehrfacher Anordnung Einrichtungen 3, 4 zur Förderung des Mediums und zur manuellen Betätigung des Austrages bzw. eine Einrichtung 6 zur Speicherung des Mediums aufweist, welches durch einen einzigen oder mehrere Medienauslässe 5 unter völliger Ablösung von der Austragvorrichtung 1 ins Freie abgegeben wird. Der Austragförderer 3 kann eine Schubkolben-Pumpe 7 sein, welche im wesentlichen im Hals 8 des Speichers 6 versenkt mit einer kappenförmigen Befestigung 9 an dem Speicher 6 befestigt ist, wenn nicht der Speichervorrat vollständig im Pumpengehäuse bzw. im Pumpenzylinder untergebracht ist. Die Vorrichtung 1 bildet einen Endteil der Einheit 2, welcher zur Betätigung des Austrages dient und den Medienauslaß 5 sowie ein diesem wenigstens teilweise vorgelagertes Arbeitsglied 10 aufweist, so daß diese Teile jederzeit nachträglich an jeder beliebigen Einheit 2 funktionsgerecht angebracht werden können, welche zur Befestigung der Teile passende Anschlußglieder aufweist.

Der Medienauslaß 5 und das Arbeitsglied 10 liegen parallel zueinander bzw. in einer gemeinsamen Achse 11, welche quer bzw. rechtwinklig zur Achse 12 der Anordnungen 2 bis 4 und 6 bis 9 vorgesehen ist, die ihrerseits etwa parallel bzw. achsgleich zueinander liegen. Am innerhalb des Speichers 6 anzuordnenden Ende weist das Pumpengehäuse der Pumpe 7 einen z.B. als Steigrohr ausgebildeten Einlaßkanal 13 auf, über welchen beim Rückhub der Pumpe 7 jeweils die Pumpkammer 14 über ein Einlaß-Einwegeventil mit Medium aus dem Speicher 6 gefüllt wird. Der entlang der Zylinderwandung der Pumpkammer 14 verschiebbare Pumpkolben ist mit einem aus dem anderen Ende des Pumpengehäuses vorstehenden Stößel 15 gegen Federkraft verschiebbar, um eine genau bestimmte Mediendosis unter Druck aus der Pumpkammer 14 durch einen Auslaßkanal 16 sowie zum und aus dem Medienauslaß 5 unter Überdruck zu fördern. Ein nach außen vorstehender Ringflansch 17 des Pumpengehäuses dient gemeinsam mit einer Dichtung 18 zur abgedichteten Anpressung des Pumpengehäuses gegen die äußere, ringförmige Stirnfläche des Halses 8 mit Hilfe der Schraub- oder Krimpkappe 9, die den Hals 8 am Außenumfang umgibt und den Austragförderer 3 gegenüber dem Speicher 6 zentriert. Der Stößel 15 bildet die einzige Befestigung für die Vorrichtung 1 bzw. das Arbeitsglied 10 oder den Kopf 20, welcher mit einer Innenhülse unter Bildung eines selbsthemmenden Klemmsitzes 19 festsitzend auf den Außenumfang des Stößels 15 parallel zur Achse 12 aufgesteckt ist. Dadurch kann jeweils zerstörungsfrei die Vorrichtung 1 von der Einheit 2 und diese vom Speicher 6 entfernt bzw. ausgewechselt werden.

Der Kopf 2 bildet mit seiner äußersten Stirnfläche eine als längliche, vertiefte Fingerkuhle ausgebildete Betätigungs-Handhabe 21 und eine zweite solche Handhabe ist, je nach den Abmessungen des Speichers 6, durch dessen Außenumfang und/oder seine von der Handhabe 21 abgekehrte Stirn- oder Bodenfläche so gebildet, daß der Gebrauch der Einheit 2 ohne weiteres mit einer einzigen Hand bewerkstelligt werden kann. Der Kopf 20 hat etwa gleiche Außenweite wie der Befestigungskörper 9, wobei demgegenüber die Außenweite des Speichers 6 größer oder etwa gleich groß sein kann. Das Arbeitsglied 10 steht jedoch über diese Außenumfänge teilweise oder im wesentlichen vollständig radial so vor, daß zwischen einer an sein Ende angelegten Oberfläche und dem Außenumfang noch ausreichend Raum für die Finger einer die Einheit 2 haltenden Hand gegeben ist.

Der Medienauslaß 5 ist durch eine feinstvernebelnde Zerstäuberdüse gebildet und als in der Achse 11 liegender feinster Durchlaß in der Stirnwand eines kappenförmigen Düsenkörpers 2 vorgesehen, der mit seinem Mantel bzw. Außenumfang festsitzend gegen die Achse 12 gerichtet bzw. radial in eine Aufnahme des Kopfes 20 eingesetzt ist. In das Kappeninnere des Düsenkörpers 22 greift ein einteilig mit dem Kopf 20 ausgebildeter Düsenkern ein, von dessen Stirnfläche die Innenseite der Stirnwand des Düsenkörpers 22 anliegt. Der Düsenkern bildet mit den inneren Umfangs- und Stirnflächen des Düsenkörpers 22 eine Dralleinrichtung 24, durch welche das Medium während des Eintretens in das innere Ende des Düsenkanales in eine Rotationsströmung um die Achse 11 versetzt wird. Die Weite des äußeren, den Medienauslaß 5 bildenden Endes dieses Düsenkanales kann wesentlich unterhalb eines oder eines halben Millimeters liegen, insbesondere wenn je Austragvorgang weniger als 0,1 bzw. 0,05 Milliliter Medium ausgetragen werden soll. Der Medienauslaß bildet zweckmäßig den kleinsten Strömungsquerschnitt des von der Pumpkammer 14 zu ihm führenden Auslaßkanales, welcher im Anschluß an den Stößel 15 im Kopf 20 einen zur Achse 11 etwa parallelen bzw. exzentrischen Zuführkanal 25 aufweist, der an das innere Ende des Düsenkörpers 22 und dadurch an die Dralleinrichtung 24 anschließt sowie auf der zur Handhabe 21 benachbarten Seite der Achse 11 liegt. Bei Betätigung tritt aus dem Medienauslaß 5 ein zerstäubter Kegelstrahl unter verhältnismäßig geringem, jedoch so großem Druck aus, daß er annähernd geradlinig das Ende des Arbeitsgliedes 10 erreicht, nicht jedoch dessen Innenumfang benetzt. Zwischen der Pumpkammer 14 und dem Medienauslaß 5 kann ein Steuerventil, z.B. ein mit der Kolbeneinheit verschiebbares Einwege- und/oder Überdruckventil vorgesehen sein oder die Medienzufuhr zum Medienauslaß 5 kann je nach den Erfordernissen ventilfrei sein, so daß der Austragdruck bzw. dessen Druck-Kennlinie nur von der manuellen Handhabung abhängt. Das Austragvolumen ist durch Anschlagbegrenzung des Pumphubes bzw. des Pumpkolbens bestimmt, welcher mit seiner vorderen Stirnfläche gegen eine innere Stirnfläche des Pumpengehäuses anschlagen kann, wonach er bei Freigabe des Kopfes 20 durch Federkraft wieder zurückbewegt wird und dabei Medium aus dem Speicher 6 ansaugt.

Das kappenförmige Arbeitsglied 10 weist im wesentlichen nur einen einzigen Mantel 26 und eine einzige Bodenwand 27 am hinteren Ende auf, während das vordere Ende des Mantels 26 eine ringförmig geschlossene Endfläche 28 bildet, die über den Innenumfang des übrigen Mantels 26 nicht vorsteht. Der scheibenförmig ebene, zur Achse 12 etwa parallele Boden 27 schließt mit seinem radial inneren Bereich einteilig so an den die Düse 23 umgebenden Bereich sowie den äußersten Mantel des Kopfes 20 an, daß er eine Fortsetzung dieses Mantels in Umfangsrichtung bildet und das Arbeitsglied 10 sowohl über die äußerste Stirnfläche bzw. Handhabe 21 des Kopfes 20 als auch über dessen davon abgekehrte Stirnfläche vorsteht. Gleichzeitig steht der Medienauslaß 5 mit einem ihn aufnehmenden, konischen Radialvorsprung über die Außenseite des Kopfmantels bzw. die Innenseite der Bodenwand 27 frei ausragend in das Arbeitsglied 10 vor und zwar um ein Maß, daß wesentlich kleiner als die Länge des Arbeitsgliedes 10 bzw. die Hälfte, ein Viertel oder ein Fünftel davon ist.

Der Mantel 26 bildet drei in Längsrichtung aneinander schließende, unterschiedliche Abschnitte 29, 30, 31, ist jedoch von der Innenseite der Bodenwand 27 bis zum Abschnitt 31 in jedem Bereich zumindest spitzwinklig erweitert. Der an die Bodenwand 27 einteilig anschließende, längste Abschnitt 29 ist über den gesamten Umfang im wesentlichen mit gleicher Steigung erweitert sowie zwei- bis dreifach länger als der Auslaßvorsprung. Der daran im Axialschnitt abgewinkelte Abschnitt 30 ist stärker und über den Umfang unterschiedlich erweitert, nämlich in zwei einander gegenüberliegenden Umfangszonen spitzwinklig und in zwei weiteren einander gegenüberliegenden Umfangszonen stumpfwinklig. Der letzte, die Endfläche 28 bildende Abschnitt 31 hat konstante Innenweite und ist wesentlich kürzer als die Abschnitte 29, 30, die etwa gleiche Wandungsdicken haben und gegenüber denen der Abschnitt 31 als verdickte Ringwulst ausgebildet ist. Die Länge der Abschnitte 29 bis 31 ist jeweils kleiner als ihre größte oder kleinste Weite.

Die Abschnitte 29, 31 sind jeweils über ihren Umfang und ihre Länge durchgehend geschlossen, während der Abschnitt 30 mit Unterbrechungen versehen ist. Zu diesem Zweck weist der Abschnitt 30 mehrere, z.B. vier gleichmäßig über seinen Umfang verteilte, fensterartige Durchbrüche 34 auf, die gemeinsam mehr als ein Viertel bzw. etwa die Hälfte des Umfanges einnehmen können und zwischen denen Längsarme 32, 33 gebildet sind, die entsprechend gleichmäßig über den Umfang verteilt sind und eine gegenüber ihrer Breite kleinere Länge haben können. Die Arme 32, 33 verbinden somit die Abschnitte 29, 31 gelenkig und können im Querschnitt entsprechend dem zugehörigen Abschnitt 30 gekrümmt sein. Der jeweilige Arm 32, 33 bildet über seine Länge einen federnden Biegestab bzw. im Anschluß an den jeweiligen Abschnitt 29, 31 ein rückfederndes Biegegelenk. Die Festigkeitsverhältnisse sind dabei so gewählt, daß die Bodenwand 27 und der Abschnitt 29 im wesentlichen nicht verformt werden, während die Abschnitte 30, 31 im Sinne einer Aufweitung und/oder einer Verengung verformt werden, jedoch der Abschnitt 31 so, daß sich sein Umfangsmaß nicht oder allenfalls nur unwesentlich durch rückfedernde Dehnung ändert.

Die Abschnitte 29 bis 31 sind in Axialansicht gleichsinnig sowie am Außen- und Innenumfang gleichermaßen quer zu den Achsen 11, 12 langgestreckt, nämlich gleichförmig elliptisch, so daß der vom Arbeitsglied 10 umschlossene Raum 36 sowie dessen in der Anlagefläche 28 liegende Endöffnung 35 entsprechende Form haben und nach außen konvex abgerundete Breitseiten 37 sowie mit kleinerem Krümmungsradius abgerundete Schmalseiten 38 der genannten Abschnitte sowie der Endfläche 28 und der Endöffnung 35 gebildet sind. Im Bereich der Breitseiten 37 liegen die demgegenüber wesentlich schmaleren Arme 32 spitzwinklig zur Achse 11, während die Arme 33 im Bereich der Schmalseiten 38 stumpfwinklig zur Achse 11 liegen. Jeweils nur ein Arm 32 bzw. 33 schließt in der Mitte der zugehörigen Seite 37, 38 einteilig an den Abschnitt 31 an, wobei die Breite der Arme 33 etwa gleich groß wie die der Schmalseite 38 sein kann. In Ansicht quer zur Achse 11 ist die Endfläche 28 im Bereich der Breitseiten 37 kontinuierlich konkav nach innen gekrümmt und im Bereich der Schmalseiten 38 konvex nach außen gekrümmt, wobei diese Bereiche mit kontinuierlichen Krümmungen ineinander übergehen.

Eine axiale Druckbelastung gegen die Endfläche 28 wird nur über die Verbindung 19 auf die Einheit 2 übertragen und führt an den Breitseiten 37 zu einer größeren radialen Aufweitung der Anlagefläche 28, des Abschnittes 31 und der Öffnung 35 als im Bereich der Schmalseiten 38, wo eine geringfügige, radial nach innen gerichtete Verengung auftreten kann, wobei jeweils die zugehörigen Arme 32, 33 entsprechende Schwenkbewegungen gegenüber den Abschnitten 29, 31 ausführen. Die genannten Bewegungen der Anlagefläche 28 werden durch deren rutschfeste Anlage auf die zugehörige Oberfläche übertragen, z.B. auf ein Augenlid, das dadurch schmerzfrei gespreitzt und offengehalten wird. Danach wird durch Bewegen der Einheit 2 gegenüber dem Kopf 20 der Förderhub ausgeführt, der zweckmäßig weniger als 10 bzw. 5 Millimeter, vorzugsweise nur etwa 2 bis 3 Millimeter beträgt. Hierdurch tritt in der Achse 11 vom hinteren Ende des Raumes 36 der Medienstrahl in Richtung zum offengehaltenen Auge aus und benetzt den Augapfel in Form eines feinstvernebelten, energiearmen Sprühstrahles sehr schonend. Zur Strömungsbeeinflussung und auch zur Beeinflussung der Ausrichtung des Augapfels kann auch in der Bodenwand 27 eine Fensteröffnung 39 vorgesehen sein, die zweckmäßig an die Oberseite des Auslaßvorsprunges und/oder den Innenumfang des Mantelabschnittes 29 anschließt, so daß das auf diese Beleuchtungszone ausgerichtete Auge gegenüber der Achse 11 schräg nach oben blickt. Hierzu sind auch die oberen Durchbrüche 34 geeignet. Eventuell an der Innenseite des Mantels 26 nach unten fließendes Medium kann sich im unteren, flachschalenförmigen Bereich der Abschnitte 29, 30, 31 bzw. des unteren Armes 32 sammeln und dort durch die Öffnung 35 hindurch leicht entfernt werden. Die größte Weite des Arbeitsgliedes 10 kann größer als seine Länge und die kleinste Weite kleiner als bzw. etwa gleich groß wie diese Länge sein.

In jedem Zustand steht das Arbeitsglied 10 mit dem größten Teil seiner Länge bzw. im wesentlichen vollständig über den Außenumfang der Einheit 2, zumindest des Befestigungskörpers 9 und des Kopfes 20 vor. Eine Ringschulter oder dgl. dieses Befestigungsgliedes 9 kann außerdem einen Anschlag 40 zur Hubbegrenzung bilden, an welchem das Arbeitsglied 10 im Bereich des Bodens 27 mit dem Außenumfang des Mantels 26 am Hubende anschlägt. Die Außenseite des Bodens 27 liegt in jeder Stellung nahe benachbart zu einer an den Anschlag 40 anschließenden Außenumfangsfläche des Körpers 9, so daß eventuelle Kippbelastungen nicht zu einer Beschädigung des Arbeitsgliedes 10 führen können, sondern dieses an der im Anschlag 40 und/oder der Umfangsfläche anschlägt und dadurch gegen weitere Verformung, insbesondere des Bodens 27 und des Abschnittes 29 gesichert ist.

Die erfindungsgemäße Austragvorrichtung kann auch allein aus dem einteiligen Arbeitsglied 10 bestehen, welches ein Befestigungsglied zur nachträglichen bzw. zerstörungsfrei leicht lösbaren Verbindung mit einem Austragkopf, dem Hals einer Tropfflasche oder dgl. aufweist. Das Befestigungsglied kann dabei mit einer hinsichtlich der Achse 11 radialen und/oder axialen Steck- bzw. Schnappverbindung zu befestigen sein, insbesondere in einer Nut, wie einer Ringnut, des Trägers 20, mit welchem das Befestigungsglied lagestarr zu verbinden ist. Ist das übrige Arbeitsglied 10 gegenüber dem Befestigungsglied bewegbar, z.B. um eine zur Achse 10 querliegende, jedoch seitlich versetzte Achse eines Filmgelenkes oder dgl. schwenkbar, so kann es aus seinem an dem Befestigungsglied mit dem Boden 27 liegenden Arbeitslage seitlich nach außen aus der Achse 11 in eine Nichtgebrauchslage herausgeschwenkt und so das Medium unabhängig vom Arbeitsglied 10 in beliebiger Entfernung zur Oberfläche aus dem Auslaß 5 ausgebracht werden. In diesem Fall weist der Boden 27 eine Durchtrittsöffnung für den Auslaßvorsprung auf, wobei das plattenförmige Befestigungsglied einteilig mit dem Arbeitsglied 10 bzw. dem Kopf 20 ausgebildet sein kann und eine federnde Schnappverbindung zur Sicherung des Arbeitsgliedes 10 in der Arbeitslage und/oder in der Nichtgebrauchslage vorgesehen ist. Das Arbeitsglied kann des weiteren als aufsteckbare Schutzkappe für den Kopf 20 bzw. die Einheit 2 vorgesehen sein und die Einheit 2 gegen unbeabsichtigte Austragbetätigung sichern.

Die Austragvorrichtung 1 weist erfindungsgemäß im Abstand außerhalb und vor der Austragdüse 5 eine ringförmige Druckfläche 28 zur Anlage am zu besprühenden Objekt, insbesondere zur Anlage um das Auge eines Patienten, auf, welche bei Druckbelastung exzentrisch aufgeweitet wird. Die Druckfläche 28 ist am Ende des Mantels 26 eines nur auf einem Teil seiner Länge elastisch nachgebenden Kappenkörpers 10 vorgesehen, welcher am Umfang und/oder am Boden von Öffnungen 34, 39 durchsetzt ist. Diese ermöglichen einen örtlich genau definiert begrenzten Lichteinfall sowie ungleichmäßige Steifigkeiten.

## Patentansprüche

1. Austragvorrichtung für Medien mit
1.1 einem Medienauslaß (5), aus dem das Medium mit einem Austragförderer (3) und einer Austragbetätigung (4) gegen einen mit dem Medium zu beaufschlagenden Teil auszutragen ist,
1.1.1 wobei dem Medienauslaß (5) ein Arbeitsglied (10) zur Zustandsbeeinflussung des mit dem Medium zu beaufschlagenden Teiles zugeordnet ist, **dadurch gekennzeichnet, daß**
1.2 das Arbeitsglied (10) elastisch verformbar und
1.2.1 als Abstandhalter für den Teil ausgebildet ist,
1.2.2 sowie am freien Ende eine in einem ringförmigen Kranz liegende langgestreckte Anlagefläche (28) für den Teil mit Schmal- und Breitseiten (38, 37) aufweist, wobei
1.3 die Anlagefläche zur Spreizung des Teils bei einer gegen den Teil gerichteten Druckbelastung im Bereich der Breitseiten (37) radial nach außen aufweitbar ausgebildet ist,
1.4 der Medienauslaß (5) durch eine Zerstäuberdüse (23) gebildet ist und
1.5 der Austragförderer (3) eine Pumpe (7) mit einer durch die Austragbetätigung (4) im Volumen zu verengenden Pumpkammer (14) ist.

2. Austragvorrichtung nach Anspruch 1, **dadurch gekennzeichnet, daß** das Arbeitsglied als Dichtglied gegenüber dem Teil, als Sammelkammer für Medium, als Beaufschlagungskammer, als Schutzschild für einen Austragstrahl sowie ggf. als Belüftungskammer und als Lichtkammer ausgebildet ist.

3. Austragvorrichtung nach Anspruch 1 oder 2, **dadurch gekennzeichnet, daß** die Anlagefläche (28) des Arbeitsgliedes (10) mit wechselnden Abständen ringförmig um die Auslaßachse (11) angeordnet ist.

4. Austragvorrichtung nach einem der vorhergehenden Ansprüche, **dadurch gekennzeichnet, daß** ein die Anlagefläche (28) bildender Anlageabschnitt (31) des Arbeitsgliedes (10) an mindestens einem biegeflexiblen Längsarm (32, 33) angeordnet ist.

5. Austragvorrichtung nach Anspruch 4, **dadurch gekennzeichnet, daß** mehrere, um die Auslaßachse (11) angeordnete Längsarme (32, 33) zum Anlageabschnitt (31) divergieren.

6. Austragvorrichtung nach einem der vorhergehenden Ansprüche, **dadurch gekennzeichnet, daß** das Arbeitsglied (10) mindestens eine mantelförmige Wandung (30) mit wenigstens einem, vorzugsweise mehreren über den Umfang verteilten Durchbrüchen (34) aufweist bzw. in einer Bodenwandung (27) des Arbeitsgliedes (10) nur oberhalb der Auslaßachse (11) ein Durchbruch (39) vorgesehen ist.

7. Austragvorrichtung nach einem der vorhergehenden Ansprüche, **dadurch gekennzeichnet, daß** das Arbeitsglied (10) napfförmig ist und mit einer ringscheibenförmigen Bodenwand (27) an einen den Medienauslaß (5) aufweisenden Austrag- und Betätigungskopf (20) einteilig anschließt, wobei das Arbeitsglied (10) zu seinem freien Ende mit unterschiedlichen Steigungswinkeln erweitert ist derart, daß es am freien, die Anlagefläche (28) bildenden Ende seine größte Weite hat.

8. Austragvorrichtung nach einem der vorhergehenden Ansprüche, **dadurch gekennzeichnet, daß** die Anlagefläche (28) in Längsansicht annähernd elliptisch ist und in Radialansicht mit gließenden Übergängen axial unterschiedlich weit vorsteht und daß um die Auslassachse (11) benachbarte Zonen der Anlagefläche (28) bei gemeinsamer axialer Druckbelastung unterschiedliche Radialauslenkungen aufweisen.

9. Austragvorrichtung nach einem der vorhergehenden Ansprüche, **dadurch gekennzeichnet, daß** das Arbeitsglied (10) eine an den Augenbereich um den Augapfel angepasste Anlagefläche aufweist.

## Claims

1. Discharge device for media, having
1.1 a media outlet (5) from which the medium is to be discharged with a discharge feeder (3) and a discharge actuator (4) against a component which is to be acted upon with the medium,
1.1.1 an operating member (10) being associated with the media outlet (5) in order to influence the state of the component to be acted upon with the medium, **characterised in that**
1.2 the operating member (10) is resiliently deformable and
1.2.1 is constructed as a spacing member for the component,
1.2.2 and has, at the free end, an elongate contact face (28) located in an annular crown for the component having narrow and wide sides (38, 37),
1.3 the contact face being constructed so as to be able to be spread radially outwards in order to widen the component in the event that pressure is applied against the component in the region of the wide sides (37),
1.4 the media outlet (5) being formed by a spray nozzle (23) and
1.5 the discharge feeder (3) being a pump (7) having a pump chamber (14) which is to be restricted in terms of volume by the discharge actuator (4).

2. Discharge device according to claim 1, **characterised in that** the operating member is constructed as a sealing member with respect to the component, as a collection chamber for media, as an actuation chamber, as a protective shield for a discharge jet, and optionally as a ventilation chamber and as a light chamber.

3. Discharge device according to claim 1 or 2, **characterised in that** the contact face (28) of the operating member (10) is arranged with varied spacing in an annular manner about the outlet axis (11).

4. Discharge device according to any one of the preceding claims, **characterised in that** a contact portion (31) of the operating member (10) that forms the contact face (28) is arranged on at least one resiliently flexible longitudinal arm (32, 33).

5. Discharge device according to claim 4, **characterised in that** a plurality of longitudinal arms (32, 33) which are arranged about the outlet axis (11) diverge towards the contact portion (31).

6. Discharge device according to any one of the preceding claims, **characterised in that** the operating member (10) has at least one cover-like wall (30) having at least one, preferably a plurality of aperture(s) (34) which are distributed over the periphery or only one aperture (39) is provided in a base wall (27) of the operating member (10) above the outlet axis (11).

7. Discharge device according to any one of the preceding claims, **characterised in that** the operating member (10) is cup-like and integrally adjoins, with an annular base wall (27), a discharge and actuation head (20) having the media outlet (5), the operating member (10) being widened towards the free end thereof with different angles of pitch in such a manner that it is widest at the free end which forms the contact face (28).

8. Discharge device according to any one of the preceding claims, **characterised in that** the contact face (28) is substantially elliptical when viewed in a longitudinal direction and, when viewed in a radial direction, protrudes axially to differing extents with smooth transitions and **in that**, about the outlet axis (11), adjacent zones of the contact face (28) have differing radial deflections when subjected to common application of pressure in an axial direction.

9. Discharge device according to any one of the preceding claims, **characterised in that** the operating member (10) has a contact face which is adapted to the eye region around the eyeball.

## Revendications

1. Dispositif de décharge pour fluides avec
1.1 un échappement de fluide (5) à partir duquel le fluide doit être déchargé avec un convoyeur de décharge (3) et une commande de décharge (4) contre une partie destinée à être alimentée avec le fluide,
1.1.1 dans lequel un élément de travail (10) est associé à l'échappement de fluide (5) pour influencer l'état de la partie destinée à être alimentée avec le fluide, **caractérisé en ce que**
1.2. l'élément de travail (10) est déformable élastiquement et
1.2.1. il est réalisé sous forme d'entretoise pour la partie,
1.2.2. et il comporte à son extrémité libre une surface d'appui (28) allongée placée dans une couronne annulaire pour la partie avec des côtés étroits et des côtés larges (38, 37), dans lequel
1.3. la surface d'appui est conçue pour s'élargir radialement vers l'extérieur pour écarter la partie en cas de charge de pression dirigée contre la partie dans la zone des côtés larges (37),
1.4. l'échappement de fluide (5) est constitué par une tubulure de pulvérisation (23) et
1.5. le convoyeur de décharge (3) est une pompe (7) avec une chambre de pompe (14) dont le volume doit être étranglé par la commande de décharge (4).

2. Dispositif de décharge selon la revendication 1, **caractérisé en ce que** l'élément de travail est conçu comme élément étanche par rapport à la partie, comme chambre collectrice pour le fluide, comme chambre d'alimentation, comme écran de protection pour un jet de décharge et, le cas échéant, comme chambre de ventilation et comme chambre intérieure.

3. Dispositif de décharge selon la revendication 1 ou 2, **caractérisé en ce que** la surface d'appui (28) de l'élément de travail (10) est disposée en forme d'anneau avec des intervalles variables autour de l'axe d'échappement (11).

4. Dispositif de décharge selon l'une quelconque des revendications précédentes, **caractérisé en ce qu'**un tronçon d'appui (31) de l'élément de travail (10) formant la surface d'appui (28) est disposé sur au moins un bras longitudinal flexible (32, 33).

5. Dispositif de décharge selon la revendication 4, **caractérisé en ce que** plusieurs bras longitudinaux (32, 33) disposés autour de l'axe d'échappement (11) divergent en direction du tronçon d'appui (31).

6. Dispositif de décharge selon l'une quelconque des revendications précédentes, **caractérisé en ce que** l'élément de travail (10) comporte au moins une paroi en forme d'enveloppe (30) avec au moins un, de préférence plusieurs, passages (34) répartis sur le pourtour ou **en ce qu'**il est prévu un passage (39) dans une paroi de fond (27) de l'élément de travail (10) uniquement au-dessus de l'axe d'échappement (11).

7. Dispositif de décharge selon l'une quelconque des revendications précédentes, **caractérisé en ce que** l'élément de travail (10) a la forme d'une cuvette et est raccordé d'un seul tenant, avec une paroi de fond en forme de disque annulaire (27), à une tête de décharge et de commande (20) munie de l'échappement de fluide (5), l'élément de travail (10) étant élargi avec différents angles d'inclinaison en direction de son extrémité libre de telle sorte qu'il atteint sa largeur maximale à l'extrémité libre formant la surface d'appui (28).

8. Dispositif de décharge selon l'une quelconque des revendications précédentes, **caractérisé en ce que**, vue longitudinalement, la surface d'appui (28) est à peu près elliptique et, vue radialement, elle fait saillie axialement sur une distance variable avec des transitions progressives et **en ce que** des zones de la surface d'appui (28) adjacentes autour de l'axe d'échappement (11) présentent des déviations radiales différentes en cas de charge de pression axiale commune.

9. Dispositif de décharge selon l'une quelconque des revendications précédentes, **caractérisé en ce que** l'élément de travail (10) comporte une surface d'appui adaptée à la zone oculaire autour du globe oculaire.
